# EUROPEAN PATENT SPECIFICATION

(11) **EP 1 388 329 B1**
(45) Date of publication and mention of the grant of the patent: **13.05.2009**
(21) Application number: 03017421.3
(22) Date of filing: 01.08.2003
(51) Int. Cl.: A61F 2/36

(54) **Femoral prosthesis for hip joint**
Femurprothese für das Hüftgelenk
Prothèse fémorale pour l'articulation de la hanche

(30) Priority: 05.08.2002 IT UD20020173
(43) Date of publication of application: 11.02.2004
(73) Proprietor: LIMA Lto SpA, 33030 Villanova di San Daniele del Friuli (IT)
(72) Inventor: Lualdi, Gabriele, 33034 Fagagna(UD) (IT); Dalla Pria, Paolo, 33100 Udine (IT)
(74) Representative: Petraz, Gilberto Luigi

(56) References cited:
- EP-A- 0 094 829
- EP-A- 0 791 342
- WO-A-91/07932
- DE-U- 9 103 574
- DE-U- 9 313 233
- FR-A- 2 057 418
- FR-A- 2 353 275
- FR-A- 2 773 469
- FR-E- 80 495
- GB-A- 764 600
- US-A- 4 846 841
- US-A- 5 888 211
- US-A- 6 096 084
- US-A1- 2001 049 561

## Description

### FIELD OF THE INVENTION

The present invention concerns a femoral prosthesis for the hip articulation, of the so-called "resurfacing" type, that is, a prosthesis suitable to be fixed on the top of a neck of a femur, leaving it substantially intact. The femoral prosthesis according to the invention comprises a head, substantially hemispherical in shape, able to be inserted into a corresponding acetabular seating of the hip, and a pin element able to be clamped in the upper part of the femur.

The femoral prosthesis according to the present invention is of the at least partly interchangeable type due to the fact that the pin element is a distinct element and does not form a single piece with the femoral head. In this way, for example, the femoral head can be replaced by another, new one of the same type or by others of a different shape and/or size, even during the pre-installation step, for example to obtain a different orientation of the femoral head with respect to the pin element.

With the femoral prosthesis according to the present invention it is also possible to vary the reciprocal distance of the head with respect to the pin element, in order to modify for example the tension exerted by the head itself along the whole femur. Moreover, the prostheses according to the present invention allows to perform the implant in two distinct steps, at a first moment inserting the pin element into the femur along the cephalic axis, and at a second moment, after having made a relative seating on the natural femoral head, applying the head.

DE 91 03 574 U discloses a femoral prosthesis having the features of the preamble of the main claim.

US-A-4.846.841 and US-A-6.096.084 disclose other examples of femoral prosthesis in which the femoral head is a distinct and removable component with respect to the pin means which are inserted into the femur.

### BACKGROUND OF THE INVENTION

A femoral prosthesis is known, the so-called resurfacing prosthesis, for the articulation of a femur in a hip, which comprises as its essential parts a metal pin, straight or arched, which is inserted and fixed in the neck of the femur, and a femoral head, made in a single piece with the pin and able to be inserted into a mating acetabular cup, previously inserted into a natural acetabular seating.

This type of prosthesis is called a resurfacing prosthesis precisely because it provides to replace only part of the femoral head, leaving the neck of the femur intact. This prosthesis, with respect to the complete prosthesis, has the advantage that it causes minimum anatomical alteration; this allows to preserve the bone tissues in favour of possible subsequent revision interventions, which can thus be carried out as if it were a normal first implant.

To use such resurfacing prostheses it is necessary that the patient should have a good quality bone tissue and minimum anatomical alteration of the femoral head.

Moreover, the diameter of the artificial femoral head must be approximately the same value as the natural head, in order to keep unchanged the anatomy of the proximal part of the femur. Since the acetabular cup wherein the head is inserted must have a high inner diameter, the acetabular cup must necessarily have a very limited thickness. Therefore, for reasons of structural resistance, in the present state of the art it is preferred not to use cups made of polyethylene, or of ceramic, which require an adequately large thickness; therefore, the whole prosthetic implant is preferably made completely of metal material, achieving a coupling through metal-metal sliding.

The metal used is preferably cobalt, or a cobalt-based alloy, because of its good resistance to the wear caused by the coupling.

A first disadvantage of resurfacing femoral prostheses of a known type is the fact that cobalt-based alloys can be integrated with the bone tissue only with difficulty; there is therefore the risk that the pin is not adequately fixed in the femur and this can create damage to the bone structure, or at least can cause its stabilizing function to be reduced.

Another disadvantage concerns the surgical technique currently adopted to apply the resurfacing femoral prosthesis of a known type. The classical surgical technique provides to prepare an outer cylindrical/conical seating made by milling the anatomical femoral head and to insert the pin into the diaphysis channel. The milling and subsequent application of the femoral head are then carried out in absence of a point of reference on the femur and, consequently, there is the possible risk of introducing the pin in an incorrect position.

Applicant has devised and embodied the present invention to overcome these shortcomings of the state of the art and to obtain other advantages.

### SUMMARY OF THE INVENTION

The present invention is set forth and characterized in the main claims, while the dependent claims describe other characteristics of the invention.

The purpose of the present invention is to obtain a femoral prosthesis of the so-called resurfacing type by means of which it is possible to selectively combine various types of prosthetic heads to a same pin, in order to optimize, on each occasion, the configuration of the prosthetic implant with regard to the real conditions of the articulation.

Another purpose of the invention is to achieve a femoral prosthesis whose head can be easily replaced without intervening on the pin attached to the end of the femur.

A further purpose is to facilitate the surgeon's work, allowing him both to have a precise reference during the installation of the head, and also to implant the prosthesis in two different steps, even distant over time.

In accordance with these purposes, in a femoral prosthesis of a hip of the so-called resurfacing type, which comprises at least a femoral head of a hemispherical shape and relative pin means, able to be engaged and clamped in a top portion of the femur, the femoral head is a distinct and removable component with respect to the pin means. According to a characteristic of the present invention, in fact, the pin means are provided with, or are associated with, coupling means able to be inserted into a mating seating of the femoral head in order to obtain a removable constraint between the latter and the pin means.

In this way, the femoral head can be removed from the acetabular seating without operating directly on the bone, and therefore with great ease.

According to the present invention, the coupling means comprise a flange-type insert able to be connected to the pin means, which is inserted and coupled in a mating cavity made in the femoral head.

According to the invention, the constraint between the flange-type insert and the femoral head is achieved by means of conical coupling. According to the invention, the flange-type insert is at least partly hollow inside and defines an inner seating for attachment to the top portion made on the femur.

Advantageously, the flange-type insert is configured to be rested on the top portion of the femur suitably prepared.

After having inserted the pin means in the top of the femur, the flange-type insert can be attached, for example hooked, cemented, or rested on the femoral seating and, at a second moment, the femoral head is anchored on said flange-type insert, for example by means of the aforesaid conical coupling. The invention allows to carry out, with the same flange-type insert already attached to the femoral seating, a plurality of trials with various femoral heads, in order to vary the head/pin distance and orientation of the prosthetic implant.

Another advantage of the present invention is that it allows to use femoral prostheses wherein the femoral head is made of a different material from the pin means.

Thanks to this, the pin means and the coupling means can be made with a material, for example titanium or titanium alloy, particularly suitable for integration in a bone tissue, while the femoral head can be made of an anti-wear material, such as for example cobalt alloy, as in resurfacing femoral prostheses of a known type, or of ceramic material of a suitable and high resistance.

### BRIEF DESCRIPTION OF THE DRAWINGS

These and other characteristics of the present invention will become apparent from the following description of a preferential form of embodiment, given as a non-restrictive example, with reference to the attached drawings wherein:
- fig. 1 shows a section view of the femoral prosthesis according to the invention assembled and fixed in an acetabular seating;
- fig. 2 shows an exploded view of the prosthesis in fig. 1;
- fig. 3 shows a variant of the prosthesis in fig. 1.

### DETAILED DESCRIPTION OF TWO PREFERENTIAL FORMS OF EMBODIMENT OF THE INVENTION

With reference to the attached drawings, a resurfacing femoral prosthesis 10 according to the present invention is attached to the top 21 of a natural femoral head and is able to replace the natural femoral head, leaving the neck of the femur 11 substantially intact.

The femoral prosthesis 10 comprises a head or cap 12, substantially hemispherical in shape, in the inner zone of which a cavity 14 is made, and a pin 18 which is inserted under pressure at the base of the top 21 of the natural femoral head.

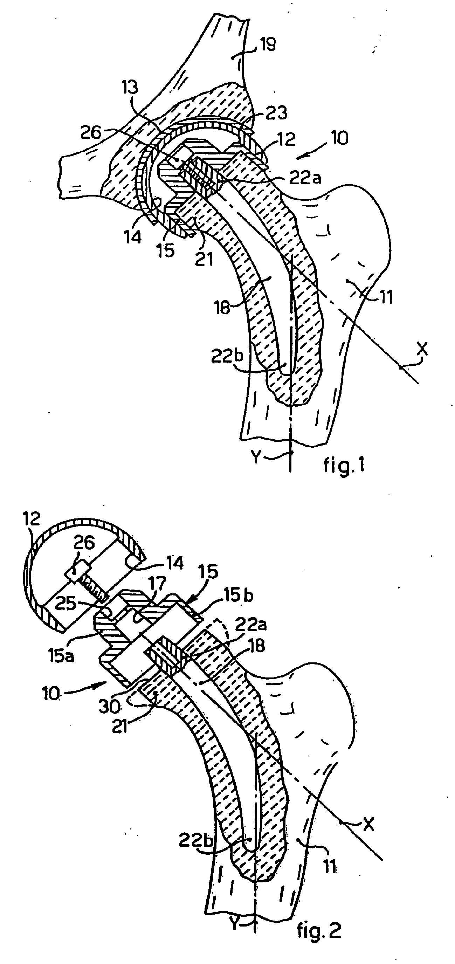

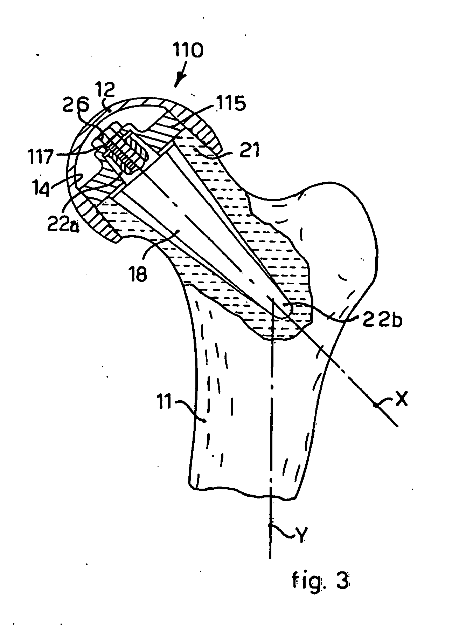

In one embodiment of the invention, the pin 18, whether it be straight (fig. 3) or arched (figs. 1 and 2), has a transverse section which is not constant over its whole length, but conical, with a greater section in correspondence with an upper end 22a towards the head 12 and smaller at the opposite end 22b, which is inserted into the neck of the femur 11. This shape of the pin 18 allows to facilitate, during the surgical operation, the insertion under pressure from above.

The head 12 is able to be inserted into an acetabular cup 23 which is fixed to a mating acetabular seating 13 of a hip 19.

The femoral prosthesis 10 according to the present invention is of the interchangeable type, due to the fact that the head 12 and the pin 18 are two distinct elements.

According to one characteristic of the present invention, a flange-type insert 15 is interposed between the head 12 and the pin 18 and is able to be inserted into the cavity 14 of the head 12 to allow a correct positioning and coupling of the two elements.

The flange-type insert 15, in figs. 1 and 2, comprises an upper coupling portion 15a, hollow inside, in which a seating 17 is made inside which the upper end 22a of the pin 18, protruding from the top 21, is inserted along a cephalic axis X and coupled through a first conical coupling.

The flange-type insert 15 also comprises a lower portion 15b, also hollow inside, which is able to be mounted, due to coupling in shape with its inner perimeter, on the top 12, so as to surround at least partially the top 21.

The lower portion 15b, which is substantially conical in shape, is able to be inserted, by means of another conical coupling with its outer perimeter, into. the cavity 14 of the head 12. The axis of the cone of this second coupling is coaxial with respect to the axis of the cone of the first coupling.

The top 21 of the natural femoral head, after being sectioned to obtain a plane surface 30 and substantially orthogonal to the cephalic axis X, is suitably milled, before the flange-type insert 15 is mounted, until an outer seating is obtained of a conical or cylindrical shape, into which the flange-type insert 15 is inserted.

Above the upper coupling portion 15a there is also a housing seating 25 for an attachment screw 26, able to clamp the flange-type insert 15 with the pin 18 in the assembled condition. The flange-type insert 15 can also be cemented on the top 21.

According to a variant shown in fig. 3, the flange-type insert 115 of the femoral prosthesis 110 is in the shape of an axially-holed disc, which is simply rested flat on the resection of the natural femoral head. In the central portion of the flange-type insert 115 a seating 117 is made of a conical or cylindrical shape, into which the upper end 22a of the pin 18 is inserted, coupled through conical coupling and fixed by means of the attachment screw 26.

In this solution, the head 12 is able to be mounted, by means of conical coupling with the perimeter of its inner cavity 14, directly on the top 21 of the femur 11, so as to couple also with the outer perimeter of the flange-type insert 115 by means of another conical coupling.

According to one characteristic, the distance between the pin 18 and the head 12 can be selectively varied using a flange-type element 15, 115 of different size. To be more exact, by varying the size of the upper coupling portion 15a with respect to the size of the lower portion 15b, or by varying the thickness of the flange-type insert 115, it is possible to obtain a variation in the distance of the head 12 from the neck of the femur 11, so as to vary, also during the step when the implant is tested, the articular tension and the offset with respect to a diaphysis axis Y of the neck of the femur 11.

By means of the femoral prosthesis 10 as described heretofore, it is also possible to modulate, with a large number of combinations, the diameter of the head 12 and the pin 18, according to the size of the neck of the femur 11 and of the acetabular seating 13, in order to obtain a prosthesis suitable to the structure and conformation of the natural femur, even in the case of a worsening of the articulation.

A pre-defined angle of the conical coupling between the flange-type insert 15, 115 and the pin 18, with respect to the other conical coupling between the head 12 and the flange-type insert 15, 115, allows to choose, with a large number of possible combinations and according to necessity, the reciprocal orientation of the three elements.

To this end, according to a first embodiment, the flange-type insert 15, 115 can be designed in such a way that the axis of the cone of one coupling is angled with respect to the axis of the cone of the other coupling.

This allows the head 12, at the moment of coupling of the flange-type insert 15, 115 and the head 12, to be already automatically oriented with a certain angle with respect to the pin 18 and hence to the neck of the femur 11.

In another embodiment, the axis of the cone of one coupling is offset and parallel with respect to the axis of the cone of the other coupling.

This allows both to automatically orientate the head 12 with respect to the pin 18 and also to translate the head 12, displacing its center in a front-rear or side-median direction with respect to the neck of the femur 11.

According to a variant, the flange-type insert 15, 115 is coupled eccentrically with the head 12.

The type of coupling between the elements is not in any case restrictive.

In a first variant, not shown in the drawings, the head 12 can be hooked to the flange-type insert 15, 115 or screwed.

According to another embodiment, the flange-type insert 15, 115 is constrained fixedly to the pin 18. In accordance with this embodiment, the flange-type insert 15, 115 is made in a single piece with the pin 18.

In another embodiment, the flange-type insert 15, 115 is cemented on the pin 18.

According to another characteristic, the head 12 is made of a different material with respect to the pin 18 and the flange-type insert 15, 115.

To be more exact, the pin 18 and the flange-type insert 15, 115 are advantageously made of titanium or a titanium alloy, since this metal is suitable for bone integration, whereas the head 12 is advantageously made of a cobalt-based alloy or other material with a great mechanical resistance.

The implant of the femoral prosthesis 10 is achieved in the following manner.

At a first moment, a resection is made of the top 21 in order to obtain the plane surface 30 substantially orthogonal to the cephalic axis X.

According to a variant, the resection of the top 21 is not perpendicular to the cephalic axis X of the neck of the femur 11, but is inclined by a certain angle with the purpose of increasing the compression forces on the femur and of reducing the shearing forces of the resection.

At a second moment, the pin 18 is inserted, approximately on the equatorial level, passing through the top 21 of the neck of the femur 11 letting its upper end 22a emerge.

Once fixed, the pin 18 can function as a guide for milling and preparing the top 21 on which the flange-type insert 15 is mounted. The milled portion is shown in fig. 2 by a line of dashes.

Subsequently, the flange-type insert 15 is mounted on the top 21, coupled and clamped to the upper end 22a of the pin 18 by means of the screw 26.

When the flange-type insert 15 is mounted, the head 12 is coupled thereon.

According to the solution in fig. 3, the flange-type insert 115 is rested on the resection of the top 21 and clamped to the upper end 22a of the pin 18. Subsequently, the head 12 is mounted on the milled portion of the top 21.

The femoral prosthesis 10 also allows to implant the prosthesis in two distinct steps, even distant over time, as in the case of the so-called two-time prosthesis application.

Two-time prosthesis application is particularly useful in the event that it is necessary to momentarily support the bone of a femur in a first step, transferring healthy bone tissue inside the femur, by means of lateral access, which is done under the greater trochanter. The transfer of bone tissue is carried out for example in interventions to fill a natural femoral head in the case of avascular necrosis thereof.

The pin 18 is introduced in this case not from the top 21, but through the lateral access and is positioned along the cephalic axis X, in such a way that the upper end 22a remains inside the natural femoral head in order to consolidate the healthy bone tissue.

Once the pin 18 has been integrated into the bone, should it be necessary to prosthetize the articulation of the hip, it is sufficient to section the upper portion of the femur, as described above, to couple the flange-type insert 15 and the head 12.

It is clear that modifications or additions may be made to the femoral prosthesis 10 as described heretofore, without departing from the field of protection of the present invention.

According to a variant, not shown in the drawings, the pin 18 is cylindrical in shape.

It is also clear that, although the description refers to a specific example, a skilled person shall be able to achieve other equivalent forms of femoral prosthesis 10, all of which shall come within the field of the present invention.

## Claims

1. Femoral prosthesis of a hip able to allow the articulation of a femur (11) in a corresponding acetabular seating (13), and comprising at least a femoral head (12) of a hemispherical shape able to be inserted into said acetabular seating (13), and pin means (18) able to be engaged and clamped in a top portion (21) of said femur (11), said femoral head (12) being a distinct and removable component with respect to said pin means (18), wherein said pin means (18) are provided with, or are associated with, a flange-type insert (15 ) inserted inside said femoral head (12) in order to obtain a removable constraint of the latter and said pin means (18), the coupling between said flangq-type insert (15 ) and the femoral head (12) being conical, **characterized in that** said flange-type insert (15) comprises a first portion (15b) able to be coupled by means of a first conical coupling in a mating seating (14) made inside said femoral head (12), said first portion (15b) being configured for resting on said top portion (21) and defining a relative hollow seating to at least partially surround said top portion (21) and attach thereto.

2. Femoral prosthesis as in claim 1, **characterized in that** said flange-type insert (15) is removable with respect to said pin means (18).

3. Femoral prosthesis as in claim 1, **characterized in that** said flange-type insert (15) comprises a second portion (15a), in which a seating (17) is made, able to allow second coupling of said flange-type insert (15) with an upper end (22a) of said pin means (18).

4. Femoral prosthesis as in claim 3, **characterized in that** said second coupling is of the conical type.

5. Femoral prosthesis as in claim 3, **characterized in that** in the upper zone of said second portion (15a) there is a housing seating (25) for an attachment screw (26) able to clamp said flange-type insert (15) in an assembled condition with said pin means (18).

6. Femoral prosthesis as in any claim from 1 to 5 , **characterized in that** said flange-type insert (15) is coupled eccentrically with said femoral head (12).

7. Femoral prosthesis as in claims 1 and 4, **characterized in that** the axis of the cone of said first coupling is coaxial with respect to the axis of the cone of said second conical coupling.

8. Femoral prosthesis as in claims 1 and 4, **characterized in that** the axis of the cone of said first coupling is angled with respect to the axis of the cone of said second conical coupling.

9. Femoral prosthesis as in claims 1 and 4, **characterized in that** the axis of the cone of said first coupling is offset and parallel with respect to the axis of the cone of said second conical coupling.

10. Femoral prosthesis as in claim 1, **characterized in that** said flange-type insert (15) is able to be cemented on said top portion (21).

11. Femoral prosthesis as in claim 1, **characterized in that** said flange-type insert (15) is made in a shingle piece with said pin means (18).

12. Femoral prosthesis as in any claim from 1 to 10 , **characterized in that** said femoral head (12) is made of a different anti-wear material with respect to said pin means (18), which are made of a material able to be easily integrated with a bone tissue.

13. Femoral prosthesis as in any claim from 1 to 12 , **characterized in that** said pin means (18), and said flange-type insert (15) are made of titanium or titanium alloy, whereas said femoral head (12) is made of a cobalt alloy, or other material with high mechanical resistance.

14. Femoral prosthesis as in any claim hereinbefore, **characterized in that** said pin means (18) are substantially conical in shape, with a lesser section at a first end (22b) on the side where it is inserted in said top portion (21), and a greater section at a second end (22a) towards said coupling means (15).

15. Femoral prosthesis as in any claim from 1 to 13 inclusive, **characterized in that** said pin means (18) are substantially cylindrical in shape.

## Patentansprüche

1. Femurprothese einer Hüfte, die eine gelenkige Lagerung des Oberschenkelknochens (11) in einer zugehörigen Hüftgelenkpfanne (13) gestattet und wenigstens einen Femurkopf (12) von einer halbkugelartigen Gestalt aufweist, der dazu eingerichtet ist, in die Hüftgelenkpfanne (13) eingefügt zu werden, und mit Schaftmitteln (18), die dazu eingerichtet sind, in einem oberen Bereich (21) des Oberschenkelknochens (11) eingefügt und befestigt zu werden, wobei der Femurkopf (12) ein separates und entfernbares Bauteil in Bezug auf die Schaftmittel (18) ist, wobei die Schaftmittel (18) mit einem flanschartigen Einsatz (15) ausgestattet oder mit diesem verbunden sind, der in den Femurkopf (12) einfügbar ist, um eine entfernbare Zwangsrelation des letzteren mit den Schaftmitteln (18) zu erhalten, wobei die Verbindung zwischen dem flanschartigen Einsatz (15) und dem Femurkopf (12) konisch ist, **dadurch gekennzeichnet, dass** der flanschartige Einsatz (15) einen ersten Abschnitt (15b) aufweist, der dazu eingerichtet ist, mittels einer ersten konischen Kupplung in eine angepasste Aufnahme (14), die innerhalb des Femurkopfes (12) ausgebildet ist, eingekuppelt zu werden, wobei der erste Abschnitt (15b) dazu eingerichtet ist, an den oberen Bereichen (21) angeordnet zu sein und eine verhältnismäßig hohle Aufnahme zu bilden, um den oberen Bereich (21) wenigstens teilweise zu umgeben und mit diesem verbunden zu sein.

2. Femurprothese nach Anspruch 1, **dadurch gekennzeichnet, dass** der flanschartige Einsatz (15) in Bezug auf die Schaftmittel (18) entfernbar ist.

3. Femurprothese nach Anspruch 1, **dadurch gekennzeichnet, dass** der flanschartige Einsatz (15) einen zweiten Abschnitt (15a) aufweist, in dem eine Aufnahme (17) ausgebildet ist, die dazu eingerichtet ist, eine zweite Verbindung des flanschartigen Einsatzes (15) mit einem oberen Ende (22a) der Schaftmittel (18) zu gestatten.

4. Femurprothese nach Anspruch 3, **dadurch gekennzeichnet, dass** die zweite Verbindung konisch ist.

5. Femurprothese nach Anspruch 3, **dadurch gekennzeichnet, dass** in dem oberen Bereich des zweiten Abschnittes (15a) ein Aufnahmeraum (25) für eine Befestigungsschraube (26) vorhanden ist, die dazu eingerichtet ist, in einer zusammengebauten Anordnung den flanschartigen Einsatz (15) mit den Schaftmitteln (18) zu verbinden.

6. Femurprothese nach einem der Ansprüche 1 bis 5, **dadurch gekennzeichnet, dass** der flanschartige Einsatz (15) exzentrisch mit dem Femurkopf (12) verbunden ist.

7. Femurprothese nach Ansprüchen 1 und 4, **dadurch gekennzeichnet, dass** die Achse des Konus der ersten Verbindung koaxial in Bezug auf die Achse des Konus der zweiten konischen Verbindung ist.

8. Femurprothese nach Ansprüchen 1 und 4, **dadurch gekennzeichnet, dass** die Achse des Konus der ersten Verbindung winklig in Bezug auf die Achse des Konus der zweiten konischen Verbindung ist.

9. Femurprothese nach Ansprüchen 1 und 4, **dadurch gekennzeichnet, dass** die Achse des Konus der ersten Verbindung versetzt und parallel in Bezug auf die Achse des Konus der zweiten konischen Verbindung ist.

10. Femurprothese nach Anspruch 1, **dadurch gekennzeichnet, dass** der flanschartige Einsatz (15) dazu eingerichtet ist, in den oberen Bereich (21) einzementiert zu werden.

11. Femurprothese nach Anspruch 1, **dadurch gekennzeichnet, dass** der flanschartige Einsatz (15) einstückig mit den Schaftmitteln (18) ausgebildet ist.

12. Femurprothese nach einem der Ansprüche 1 bis 10, **dadurch gekennzeichnet, dass** der Femurkopf (12) in Bezug auf die Schaftmittel (18), die aus einem Material hergestellt sind, das einfach in ein Knochengewebe integrierbar ist, aus einem unterschiedlichen verschleißfesten Material hergestellt ist.

13. Femurprothese nach einem der Ansprüche 1 bis 12, **dadurch gekennzeichnet, dass** die Schaftmittel (18) und der flanschartige Einsatz (15) aus Titan oder einer Titanlegierung hergestellt sind, während der Femurkopf (12) aus einer Kobaltlegierung oder einem anderen Material mit einer hohen mechanischen Widerstandsfähigkeit hergestellt ist.

14. Femurprothese nach einem der vorangehenden Ansprüche, **dadurch gekennzeichnet, dass** die Schaftmittel (18) im Wesentlichen konisch ausgestaltet sind, und zwar mit einem kleineren Abschnitt an einem ersten Ende (22b) auf der Seite, an der es in den oberen Bereichen (21) eingefügt ist, und mit einem größeren Abschnitt an einem zweiten Ende (22a) in Richtung der Verbindungsmittel (15).

15. Femurprothese nach einem der Ansprüche 1 bis 13, **dadurch gekennzeichnet, dass** die Schaftmittel (18) im Wesentlichen zylinderförmig ausgebildet sind.

## Revendications

1. Prothèse fémorale de la hanche apte à permettre l'articulation du fémur (11) dans un siège acétabulaire (13) correspondant, et comprenant au moins une tête fémorale (12) de forme hémisphérique pouvant être insérée dans ledit siège acétabulaire (13), et un moyen de brochage (18) pouvant être engagé et bloqué dans une partie haute (21) dudit fémur (21), ladite tête fémorale (12) étant un composant distinct et amovible par rapport audit moyen de brochage (18), lequel est pourvu de, ou est associé à, un insert de type collerette (15) inséré dans ladite tête fémorale (12) en vue d'obtenir une contrainte amovible de cette dernière et dudit moyen de brochage (18), l'accouplement entre ledit insert de type collerette (15) et la tête fémorale (12) étant conique, **caractérisée en ce que** ledit insert de type collerette (15) comprend une première partie (15b) pouvant s'accoupler, au moyen d'un premier accouplement conique, à un siège apparié (14) ménagé à l'intérieur de ladite tête fémorale (12), ladite première partie (15b) étant configurée pour reposer sur ladite partie haute (21) et définir un siège creux associé afin d'entourer au moins partiellement ladite partie haute (21) et de s'y fixer.

2. Prothèse fémorale selon la revendication 1, **caractérisée en ce que** ledit insert de type collerette (15) est amovible par rapport audit moyen de brochage (18).

3. Prothèse fémorale selon la revendication 1, **caractérisée en ce que** ledit insert de type collerette (15) comprend une seconde partie (15a), dans laquelle est ménagé un siège (17) apte à permettre un second accouplement dudit insert de type collerette (15) avec une extrémité supérieure (22a) dudit moyen de brochage (18).

4. Prothèse fémorale selon la revendication 3, **caractérisée en ce que** ledit second accouplement est de type conique.

5. Prothèse fémorale selon la revendication 3, **caractérisée en ce que** dans la zone supérieure de ladite seconde partie (15a) se trouve un siège récepteur (25) destiné à recevoir une vis de fixation (26) apte à bloquer ledit insert de type collerette (15) en position d'assemblage avec ledit moyen de brochage (18).

6. Prothèse fémorale selon l'une quelconque des revendications 1 à 5, **caractérisée en ce que** ledit insert de type collerette (15) présente un accouplement excentrique avec ladite tête fémorale (12).

7. Prothèse fémorale selon les revendications 1 et 4, **caractérisée en ce que** l'axe du cône dudit premier accouplement est coaxial à l'axe du cône dudit second accouplement conique.

8. Prothèse fémorale selon les revendications 1 et 4, **caractérisée en ce que** l'axe du cône dudit premier accouplement forme un angle avec l'axe du cône dudit second accouplement conique.

9. Prothèse fémorale selon les revendications 1 et 4, **caractérisée en ce que** l'axe du cône dudit premier accouplement est décalé et parallèle à l'axe du cône dudit second accouplement conique.

10. Prothèse fémorale selon la revendication 1, **caractérisée en ce que** ledit insert de type collerette (15) peut être cimenté sur ladite partie haute (21).

11. Prothèse fémorale selon la revendication 1, **caractérisée en ce que** ledit insert de type collerette (15) est réalisé d'un seul tenant avec ledit moyen de brochage (18).

12. Prothèse fémorale selon l'une quelconque des revendications 1 à 10, **caractérisée en ce que** ladite tête fémorale (12) est constituée d'un matériau anti-usure différent de celui dudit moyen de brochage (18), lequel est constitué d'un matériau pouvant s'intégrer facilement à un tissu osseux.

13. Prothèse fémorale selon l'une quelconque des revendications 1 à 12, **caractérisée en ce que** ledit moyen de brochage (18) et ledit insert de type collerette (15) sont en titane ou en alliage de titane, tandis que ladite tête fémorale (12) est en alliage de cobalt, ou constituée d'un autre matériau à haute résistance mécanique.

14. Prothèse fémorale selon l'une quelconque des revendications précédentes, **caractérisée en ce que** ledit moyen de brochage (18) est de forme sensiblement conique, la petite section se trouvant à une première extrémité (22b) du côté où il est inséré dans ladite partie haute (21), et la grande section se trouvant à une seconde extrémité (22a) vers ledit moyen d'accouplement (15).

15. Prothèse fémorale selon l'une quelconque des revendications 1 à 13 incluse, **caractérisée en ce que** ledit moyen de brochage (18) est de forme sensiblement cylindrique.
